# EUROPEAN PATENT SPECIFICATION

(11) **EP 3 598 902 B1**
(45) Date of publication and mention of the grant of the patent: **13.12.2023**
(21) Application number: 19191883.8
(22) Date of filing: 06.08.2015
(51) Int. Cl.: A23L 33/15, A23L 33/16, A23L 33/105, A61K 31/047, A61K 31/4415, A61K 31/525, A61K 31/714, A61K 35/74, A61P 3/12, A61K 35/745, A61K 35/747, A61P 3/10, A61P 5/50

(54) **VITAMIN B2 AND ITS USE**
VITAMIN B2 UND DESSEN VERWENDUNG
VITAMINE B2 ET SON UTILISATION

(30) Priority: 08.08.2014 EP 14180396; 08.08.2014 EP 14180401; 05.06.2015 EP 15170906
(43) Date of publication of application: 29.01.2020
(62) Divisional of application: 15766404.6
(73) Proprietor: Société des Produits Nestlé S.A., 1800 Vevey (CH)
(72) Inventor: SILVA ZOLEZZI, Irma, 276750 Singapore (SG); MACE, Catherine, 1093 La Conversion (CH); MINEHIRA CASTELLI, Kaori, 1804 Corsier-sur-Vevey (CH); BAKER, Philip Newton, Leicester, LE7 9GE (GB); GODFREY, Keith Malcolm, Ashurst, Hampshire SO40 7AP (GB); CHONG, Yap Seng, 268431 Singapore (SG)
(74) Representative: Kamibayashi, Lynne

(56) References cited:
- EP-A1- 0 925 790
- WO-A2-2008/071790
- CN-A- 101 371 837
- US-A1- 2002 155 163
- US-A1- 2014 161 878
- MARINE L. CROZE ET AL: "Chronic treatment with myo-inositol reduces white adipose tissue accretion and improves insulin sensitivity in female mice", THE JOURNAL OF NUTRITIONAL BIOCHEMISTRY, vol. 24, no. 2, 1 February 2013 (2013-02-01), pages 457-466, XP055212044, ISSN: 0955-2863, DOI: 10.1016/j.jnutbio.2012.01.008

## Description

### Field of the invention

The invention relates to a composition comprising vitamin B2, vitamin D and zinc for use in treating or preventing an impaired glucose tolerance in a subject.

### Background

Glucose a key nutrient which gives vital energy to living cells in our organs. The glucose can be derived from food and also de novo synthesized by the liver and kidney during a fasting condition. During digestion carbohydrates from glucidic foods are transformed into glucose that is passed through the intestinal wall into the bloodstream. This influx of glucose into the bloodstream results in an increase in the blood glucose level.

The level of blood glucose is tightly regulated by a hormone, insulin. As the blood glucose level increases insulin is secreted by the pancreas. Insulin is the hormone responsible for bringing glucose to insulin-sensitive tissues such as liver, muscle and adipose tissues, thus lowering the blood glucose level in the blood. However, some subjects do not respond adequately to insulin.

These subjects have an insulin resistance or a low insulin sensitivity. This may be because insulin receptors in the insulin-sensitive tissues do not respond adequately to insulin. In response to an inadequate response to insulin the pancreas may secrete more insulin to compensate. These subjects maybe considered as having an impaired glucose tolerance (hereinafter IGT).

Eventually, the pancreas may fail to keep up with the body's increased need for insulin, leading to type II diabetes. According to the World Health Organization, at least 171 million people worldwide suffer from type II diabetes. Its incidence is increasing rapidly, and it is estimated that by 2030, this number will almost double. An IGT (also referred to as prediabetes) is even more common, and more than one out of three adults have been estimated "prediabetic" or as having an IGT in the United States.

An IGT and/or type II diabetes are associated with an increased risk of a variety of diseases for example Cardio Vascular Disease (hereinafter CVD). CVD accounts for approximately 20% of all annual worldwide deaths, and remains one of the leading causes of death in both the developed and developing world.

Dietary and lifestyle changes, including healthier dietary habits and increased exercise, can be very efficient in improving an IGT and in preventing type II diabetes, however, patient compliance can be problematic. Drugs such as biguanides and thiazolidinediones are available for improving insulin sensitivity and/or an IGT or type II diabetes. However, many of these have unwanted side effects. Moreover, there is no practice of giving such drugs to prevent IGT.

Compositions for use in the treatment of diabetes and insulin resistance are known from e.g., CN 101371837 A, which discloses such compositions comprising B vitamins. A composition comprising vitamin C, glutamic acid and/or glutamine, cysteine, riboflavine, succinic acid, fumaric acid, coenzyme Q10 and niacin, and its use to reduce blood glucose levels, is disclosed in WO 2008/071790 A2.

Accordingly, there is a need to find alternative ways to improve insulin sensitivity and to treat or prevent an IGT and/or type II diabetes, and/or to prevent a condition associated with any of the foregoing in a subject.

The inventors have surprisingly found that vitamin B2 may be used to enhance glucose uptake in mammalian cells, in particular in mammalian muscle cells. Accordingly, administering vitamin B2 to a subject may be used to improve insulin sensitivity and/or to treat or prevent an IGT and/or type II diabetes and/or to prevent CVD and/or a condition associated with a low insulin sensitivity and/or any of the foregoing in a subject.

### Summary of the Invention

The invention is set out in the claims. A composition comprising vitamin B2, vitamin D and zinc, is used to treat or prevent an IGT in a subject e.g. a mammal such as a human, cat, or dog The present invention relates to a composition comprising vitamin B2, vitamin D and zinc for use to treat or prevent impaired Glucose Tolerance (IGT), wherein the administration of said composition is not in-utero or through breastfeeding.

A low insulin sensitivity and/or and IGT and/or type II diabetes and/or CVD are associated with an increased risk of a variety of conditions, for example strokes, circulatory problems that in extreme cases can lead to amputation, diabetic retinopathy, kidney failure, hearing loss, and impaired cognitive ability. By improving insulin sensitivity and/or treating or preventing an IGT and/or type II diabetes and/or preventing CVD, vitamin B2, or a composition comprising vitamin B2, may also be used to treat or prevent associated conditions such as those listed in a subject.

Vitamin B2 may be particularly effective, at improving insulin sensitivity and/or treating or preventing an IGT and/or type II diabetes and/or preventing CVD in a subject, when used in combination with myo-inositol (cis-1,2,3,5-trans-4,6-cyclohexanehexol) and/or one or more probiotic, and/or one or more of vitamin B6, B12 and D and/or zinc. Accordingly, a composition comprising vitamin B2 and myo-inositol (cis-1,2,3,5-trans-4,6-cyclohexanehexol) and/or one or more probiotic, and/or one or more of vitamin B6, B12 and D may be particularly effective at improving insulin sensitivity and and/or treating or preventing an IGT and/or type II diabetes and/or preventing CVD in a subject. In particularly vitamin B2 may be particularly effective when used according to the present invention in combination with vitamin D or vitamin D and zinc.

The vitamin B2, the vitamin D and the zinc, and optionally myo-inositol and/or one or more probiotics and/or one or more of vitamin B6, and vitamin B12, comprised in a composition, may be administered enterally to a subject.

The vitamin B2, the vitamin D and the zinc, and optionally myo-inositol and/or one or more probiotics and/or one or more of vitamin B6, and vitamin B12, may be administered or employed in any form suitable for ingestion by a subject e.g. in the form of a composition for example a nutritional product, a food product, a functional food product, a healthy ageing product, a dairy product, a nutritional supplement, a pharmaceutical formulation, a beverage product, or a pet food product.

A composition comprising vitamin B2, vitamin D and zinc, and and any optional ingredients defined herein, may be provided along with a label indicating dosage requirements in a kit for use to treat or prevent an IGT in a subject.

### Drawings

FIG. 1 - Charts illustrating the effect of vitamin B2, in the absence of insulin, on glucose uptake in L6 differentiated muscle cells after 2 hour and 4 hour incubations.
FIG. 2 - Chart illustrating the effect of a combination of vitamin B2 and 1,25-dihydroxyvitamin D3, in the absence of insulin, on glucose uptake in L6 differentiated muscle cells after 2 hour and 4 hour incubations.
FIG. 3 - Chart illustrating the effect of the combination of vitamin B2 and 1,25-dihydroxyvitamin D3 and zinc, in the absence of insulin, on glucose uptake in L6 differentiated muscle cells after a 2 hour incubation.

### Detailed Description

The present invention is defined by the claims.

The term "subject" as used herein refers to a mammal and more particularly a cat, a dog or a human. The human may be an adult, child or baby including a preterm baby.

In a particular embodiment the subject is not a mammal that is trying to get pregnant, pregnant, or lactating.

Whether or not a mammal has an IGT or has type II diabetes may be determined by measuring its fasting glucose and/or HbAlc concentration, or by carrying out an oral glucose tolerance test (OGTT). The skilled person will be familiar with these tests and the criteria for diagnosing an IGT or type II diabetes. For humans the criteria for diagnosing an IGT or type II diabetes has been set out by the Standards of medical care in diabetes (2014) from the American Diabetes Association. A human subject is considered as having an IGT (or prediabetic condition) if their fasting plasma-glucose concentration equates to 5.6 mmol/L or more, or if their fasting HbA1C level are between 5.7-6.4 %, or blood glucose level ranges between 7.8 -11.0 mmol/L 2 hours after a 75gram glucose drink. A human subject is considered as having type II diabetes if their fasting plasma-glucose concentration equates to 7.0 mmol/L or more, or fasting HbAlc is higher than 6.5 %, or higherthan 11.1 mmol/L 2 hours after a 75gram glucose drink.

The term "treat" as used herein also encompasses amelioration and/or alleviation of a condition i.e. the amelioration and/or alleviation of the symptoms of a condition. It may for example encompass the reduction of the severity of a condition in a subject.

The term "prevent" as used herein refers to the prevention of the occurrence, or reduction of the risk of the occurrence, of a condition in a subject.

Any source or form of Vitamin B2 suitable for ingestion by a subject may be used. In particular vitamin B2 sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

According to the present invention, vitamin B2, vitamin D and zinc are used in combination with myo-inositol and/or one or more of vitamin B6, B12 and/or one or more probiotic.

A combination of vitamin B2 with myo-inositol (cis-1,2,3,5-trans-4,6-cyclohexanehexol) and/or one or more probiotic and/or one or more of vitamin B6, B12 and D may be particularly effective for improving insulin sensitivity and/or treating and/or preventing type II diabetes and/or preventing CVD and/or a condition associated with a low insulin sensitivity and/or any of the forgoing in a subject. The combination of vitamin B2 with one or more of these listed ingredients may provide an improved effect i.e. a more than additive effect or synergistic effect, over what could be expected on the basis of the effect of the individual ingredients used alone to improve insulin sensitivity and/or to treat or prevent an IGT and/or type II diabetes and/or to prevent CVD and/or conditions associated with a low insulin sensitivity and/or any of the foregoing in a subject.

Any source or form of myo-inositol suitable for ingestion by a subject may be used in the invention.

The term myo-inositol as used herein refers to myo-inositol (*cis*-1,2,3,5-*trans*-4,6-cyclohexanehexol) and /or a metabolite thereof.

A metabolite of myo-inositol can be selected from the group consisting of D-chiro-inositol and L-chiro-inositol. In particular the metabolite is D-chiro-inositol.

Any source or form of vitamins B6, B12 and D suitable for ingestion by a subject may be used in the invention.

The term vitamin B12 as used herein refers to vitamin B12 and /or a metabolite thereof.

The term vitamin B6 as used herein refers to vitamin B6 and /or a metabolite thereof.

The term vitamin D as used herein refers to vitamin D, a precursor thereof, and /or a metabolite thereof.

The term precursor as used herein refers to any substance administered e.g. ingested by a subject and used by the body of said subject to produced and/or form a particular compound.

In particular, the vitamin B6 may be pyroxidine hydrochloride, and/or a metabolite of vitamin B6 selected from the group consisting of Pyridoxal 5'-phosphate (hereinafter PLP).

In particular, the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, and 1,25-dihydroxyvitamin D2, and a combination of thereof.

More particularly the vitamin D is vitamin D3, a metabolite of vitamin D selected from the group consisting of 25-hydroxyvitamin D3, 1,25-dihydroxyvitamin, and combination thereof. Even more particularly the vitamin D3 is Cholecalciferol.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

The one or more probiotic bacteria may be any lactic acid bacteria, Bifidobacteria, or combination thereof, wherein said lactic acid bacteria and Bifidobacteria have established probiotic characteristics. Non limiting examples of lactic acid bacteria strains include; Lactobacillus rhamnosus ATCC 53103 obtainable inter alia from Valio Oy of Finland under the trade mark LGG and Lactobacillus rhamnosus CGMCC 1.3724. None limiting examples of Bifidobacteria strains include; Bifidobacterium lactis CNCM I-3446 sold inter alia by the Christian Hansen company of Denmark under the trade mark Bb12, Bifidobacterium longum ATCC BAA-999 sold by Morinaga Milk Industry Co. Ltd. of Japan under the trade mark BB536, the strain of Bifidobacterium breve sold by Danisco under the trade mark Bb-03, the strain of Bifidobacterium breve sold by Morinaga under the trade mark M-16V and the strain of Bifidobacterium breve sold by Institut Rosell (Lallemand) under the trade mark R0070.

In a particular embodiment the one or more probiotic is a mixture of Lactobacillus rhamnosus CGMCC 1.3724 and Bifidobacterium lactis CNCM I-3446
The Lactobacillus rhamnosus CGMCC 1.3724 and Bifidobacterium lactis CNCM I-3446 can be used in a ratio of 1:99 to 99:1, however, more particularly they will be used in a ratio of 1:2 to 2 to 1, even more particularly 1:1.

In particular the inventors have found that when vitamin B2 is used in combination with vitamin D there may be an improved enhancement in glucose uptake in comparison to when vitamin B2 or vitamin D is used alone. More particularly still, the inventors have found that when vitamin B2 is used in combination with vitamin D and zinc, there may be an improved enhancement in glucose uptake in comparison to when vitamin B2, zinc, or vitamin D is used alone, and that this improved enhancement may be more than additive.

The vitamin B2, the vitamin D and the zinc, is optionally in combination with e.g. the myo-inositol and/or one or more probiotic, and/or one or more of vitamin B6, and vitamin B12, can be employed in any effective dose that provides a benefit with respect to improving insulin sensitivity and/or the treatment or prevention of an IGT and/or type II diabetes.

An effective dose may be any dose that improves, by any degree, an IGT in a subject.

It is well within the purview of the skilled person to determine an effective dose. An effective dose may, for example, be determined by testing the effect of a dose on a subject's fasting glucose plasma concentration, or fasting HbAlc concentration, or by carrying out an OGTT. An effective dose should improve a subject's fasting glucose plasma concentration and/or HbAlc concentration, and/or lower a subject's glycemic response.

Typically, an effective dose will depend on the type, age, size and health status of the subject, on the subject's lifestyle, as well as on its genetic heritage.

A particularly useful dose of vitamin B2 may be 0.14 to 14 mg, 1 to 2.5mg, 1.5 to 2mg, or 1.8mg. A particularly useful dose of myo-inositol, if present, is from 0.2 to 5mg, 1.5 to 5mg, 2 to 4g, or 2g.

A particularly useful dose of the one or more probiotic, if present, is from 10e5 to 10e12 colony forming units (cfu), or 10e7 to 10e11 cfu.

A particularly useful dose of vitamin B6, if present, is 0.19 to 19 mg, or 2.6mg. A particularly useful dose of vitamin B12, if present, is 0.26 to 26µg, or 5.2µg. A particularly useful dose of vitamin D, is 1.5 to 100 µg, or 10µg. A particularly useful dose of zinc is 1.1 to 40 mg zinc.

The term "dose" as used herein refers to a daily quantity that is administered to a subject. The daily quantity or dose may be administered all at once or it may be spread out over several administrations throughout a day. The dose may be by administered by any known method, in particularly enterally e.g. orally.

The dose(s) may be administered at any time e.g. day time or night time. The dose may be particularly effective if administered before the subject has food or beverage e.g. has a meal or a snack.

In an embodiment the dose is spread over 2 administrations, in a particular the dose is spread over 2 administrations, one in the morning and one in the evening, in particularly before breakfast and before the evening meal.

The term "breakfast" as used herein refers to the first meal of the day.

The term "evening meal" as used herein refers the last meal of the day.

In a particular embodiment the administration is not in-utero or through breastfeeding.

In another aspect of the present invention there is provided a kit for use to treat or prevent an IGT in a subject, the kit comprising:
a. vitamin B2, vitamin D and zinc, optionally in combination with myo-inositol and/or one or more probiotic, and/or one or more of vitamin B6, and B12
b. A label indicating dosage requirements for a subject.

The dosage requirements may be 2 administrations per day, in particular 2 administrations per day before any food or beverage. More particularly, 2 administrations per day wherein one is in the morning and one is in the evening. Even more particularly, 2 administrations per day wherein one is in the morning before breakfast and one is in the evening before the evening meal.

The vitamin B2, vitamin D and the zinc, and optionally myo-inositol, one or more probiotic, and one or more of vitamin B6, and vitamin B12 may all be provided in a format providing sustained release of said vitamins or one or more probiotic. This way, these compounds can be consumed less frequently, while the body is still constantly supplied with sufficient amount them.

Vitamin B2, vitamin D and zinc, when used in combination with myo-inositol and/or one or more probiotic and/or one or more vitamin B6, and vitamin B12, may be administered simultaneously e.g. in the same composition as said myo-inositol and/or one or more probiotic and/or one or more of vitamin B6, and vitamin B12. Alternatively, one or more of said myo-inositol and/or one or more probiotic and/or one or more of vitamin B6, and vitamin B12 may be administered separately to said vitamin B2, vitamin D and zinc e.g. vitamin B2, vitamin D and zinc (optionally with myo-inositol, and one or more of vitamin B6, and vitamin B12) may be administered separately to said one or more probiotic. The separate administration may be sequential or simultaneously but separate administration e.g. vitamin B2 (optionally with myo-inositol, and one or more of vitamin B6, and B12) may be administered at the same time but separately to said one or more probiotic e.g. in two tablets. The vitamin B2, vitamin D and zinc (optionally with myo-inositol, and one or more of vitamin B6, and B12) may for example be administered separately and consecutively. in quick succession e.g. within 5, 4, 3, 2, 1 minute, to said one or more probiotic.

Vitamin B2, vitamin D and zinc, optionally in combination with the myo-inositol and/or one or more probiotic, and/or one or more of vitamin B6, and B12, and any other optional ingredients as set out herein, may be administered or employed in any form suitable for ingestion by the subject. They may for example be employed in the form of a composition e.g. a nutritional product, a food product, a functional food product, a healthy ageing product, a dairy product, a nutritional supplement, a pharmaceutical formulation, a beverage product, a diet, or a pet food product.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual. The term "healthy ageing product" as used herein, can refer to a diet or nutritional supplement that is intended as a means to extend lifespan in an individual. Such a product may additionally contain antioxidants or other compounds such as oxytocin, insulin, human chorionic gonadotropin (hCG), erythropoietin (EPO), dietary fiber, plant sterols, PUFA, and combinations thereof.

The term "antioxidants" as used herein refers to molecules capable of slowing or preventing the oxidation of other molecules. Preferably, antioxidants are selected from: beta-carotene, vitamin C, vitamin E, selenium, carotenoids, coenzyme Q10, flavonoids, glutathione, lutein, lycopene, polyphenols, vitamin A, vitamin B1, vitamin B6, vitamin B12, vitamin C, vitamin D, vitamin E, zeaxanthin, lipoic acid, carnosine, N-acetylcysteine, or combinations thereof.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art.

The term "beverage product" as used herein, refers to a nutritional product in liquid or semiliquid form that may be safely consumed by an individual.

The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

Vitamin B2, vitamin D and zinc, optionally in combination with the myo-inositol and/or one or more probiotic, and or one or more of vitamin B₆, and B12, may also be used in combination with other vitamins and minerals. e.g. calcium, magnesium, phosphorus, iron, zinc, copper, iodine, selenium, vitamin A or retinol activity equivalent (RAE) e.g. beta carotene or a mix of carotenoids, Vitamin C, Vitamin B1, niacin, folic acid, biotin, Vitamin E.

It may be particularly beneficial if vitamin B2, vitamin D and zinc, optionally in combination with the myo-inositol and/or one or more probiotic, and or one or more of vitamin B6, and B12, is used in combination with one or more of the following micronutrients in the following amounts: 100 to 2500 mg calcium, 35 to 350 mg magnesium, 70 to 3500 mg phosphorus, 2.7 to 45 mg iron, , 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg vitamin C, 0.14 to 14 mg vitamin B1, 1.8 to 35 mg niacin, 60 to 1000 µg folic acid, 3 to 300 µg biotin, 1.9 to 109 µg Vitamin E.

Vitamin B2, vitamin D and zinc, optionally in combination with the myo-inositol and/or one or more probiotic, and or one or more vitamin B6, and B12, may also be advantageously used in combination with at least one oligosaccharide and/or at least one long chain polyunsaturated fatty acids, such as arachidonic acid (ARA), eicosapentaenoic acid (EPA) and/or docosahexaenoic acid (DHA). The oligosaccharides and long chain polyunsaturated fatty acids can be used in any concentration safe for administration to the subject.

Vitamin B2, vitamin D and zinc, optionally in combination with the myo-inositol and/or one or more probiotic, and or one or more of vitamin B6, and B12, may also be used in combination with other ingredients commonly used in the form of composition in which it is employed e.g powdered nutritional supplement, a food product, or a dairy product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavor agents, osmotic agents, pharmaceutically acceptable carriers, preservatives, sugars, sweeteners, texturizers, emulsifiers, water and any combination thereof.

However, notwithstanding the above, a high fat intake can be undesirable and is linked to an increased risk of excess weight and obesity. Accordingly, in a particular embodiment the vitamin B2, vitamin D and zinc, optionally in combination with the myo-inositol and/or one or more probiotic, and or one or more of vitamin B6, and B12 is not used in combination with a long chain polyunsaturated fatty acid and/or animal fats and/or vegetable fats.

In another particular embodiment the vitamin B2, vitamin D and zinc, optionally in combination with the myo-inositol and/or one or more probiotic, and/or one or more of vitamin B6, and B12

is not used in combination with an oligosaccharide.

In particular the inventors have found that when vitamin B2 is used in combination with vitamin D there may be an improved enhancement in glucose uptake in comparison to when vitamin B2 or vitamin B is used alone. More particularly still, the inventors have found that when vitamin B2 is used in combination with vitamin D and zinc, there may be an improved enhancement in glucose uptake in comparison to when vitamin B2, zinc, or vitamin D is used alone, and that this improved enhancement may be more than additive.

According to the invention vitamin B2 is used in combination with zinc and vitamin D.

As stated hereinabove, a low insulin sensitivity, and IGTand/or type II diabetes in a subject is associated with a variety of conditions that affect the subject e.g. CVD. A low insulin sensitivity, and IGT and/or type II diabetes is considered as being associated with a condition if it increases the risk of a subject having or developing that condition during its lifetime.

Non limiting examples of conditions associated with a low insulin sensitivity and/or type II diabetes include: CVD, strokes, circulatory problems that in extreme cases can lead to amputation, diabetic retinopathy, kidney failure, hearing loss, and impaired cognitive ability.

A low insulin sensitivity, an IGT, and type II diabetes are more prevalent amongst subjects who are overweight or obese, or if there is a family history of type II diabetes.

Accordingly, vitamin B2, vitamin D and zinc, optionally in combination with the myo-inositol and/or one or more probiotic, and/or one or more of vitamin B6, and B12, may be particularly beneficial for improving a low insulin sensitivity and/or treating and/or preventing an IGT, and/or type II diabetes and/ or preventing CVD and/or conditions associated with a low insulin sensitivity and/or any of the foregoing in a one or more of these subsets of subjects. The invention relates to the treatment and/or prevention of an IGT.

The present invention will now be described in further details by the way of the following examples.

### Examples

### Example 1

An example composition comprising vitamin B2 in combination with myo-inositol, vitamins B6, B12 and D, and Lactobacillus rhamnosus GG¹ and Bifidobacterium lactis BB12² is set out in table 1.

The composition in table I is for a nutritional supplement in a powder form, intended to be sprinkled on food.

**Table 1 : Composition of Example 1**

| **Ingredient** | **Amount per daily dose** |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG¹⁾ | 1x10⁹ cfu |
| Bifidobacterium lactis BB12²⁾ | 1x10⁹ cfu |

| | |
|---|---|
| 1) Strain deposited as CGMCC 1.3724 2) Strain deposited as CNCM I-3446 | |

The composition may be provided as a kit of parts comprising in one sachet the probiotic as a powder and in a second sachet all other ingredients.

### Example 2

A milk powder drink for a human subject to be reconstituted in water is provided consisting of 30g of milk powder per serving admixed with the ingredients listed in Table 1, in half of the amounts mentioned in Table 1. The composition is to be administered to a human subject twice per day.

### Example 3

The effect of vitamin B2 on glucose uptake in L6 differentiated muscle cells was measured after short (2 hrs) and long term (24 hrs) incubations.

Ingredient G refers to vitamin B2, ingredient k refers to 1,25-dihydroxyvitamin D3, and ingredient J refers to zinc chloride.

Ingredient G was tested at 1 µM. Ingredient K was tested at 10 nM, and ingredient J was tested at 20 µM.

The day of the experiment, the ingredients were diluted in a serum-free medium (DMEM) containing 5.5 mM D-glucose to achieve the final tested concentrations and 0.1 % of DMSO.

L6 myoblasts were maintained in growth medium or DMEM 25 mM glucose supplemented with 10 % of fetal bovine serum (FBS), 100 U/ml penicillin, and 100 µg/ml streptomycin. For the glucose uptake study, cells were grown on 24 well plates at a density of 1000-1500 cells/well in 0.5 mL of growth medium. Five days after plating, the differentiation induction into myotubes was carried out in DMEM 25 mM glucose containing 2 % FBS, 100 U/ml penicillin, and 100 µg/ml streptomycin at 37°C under a 5 % CO₂ atmosphere. The culture medium was changed every other day.

For short term incubation (2 hrs), the day prior to the test, the culture medium was replaced by DMEM 25 mM D-glucose containing 0.25 % BSA, 100 U/ml penicillin, and 100 µg/ml streptomycin. The day after and prior to the test, cells were washed and incubated in DMEM containing 5.5 mM D-glucose, 0.25 % BSA in the presence of the ingredients for 2 hours without insulin.

For long term incubation (24 hrs), the day prior to the test, cells were pre-incubated in the presence of the tested ingredients (ingredient J, K, or a combination thereof) for 22 hours in DMEM 25 mM D-glucose containing 0.25 % BSA, 100 U/ml penicillin, and 100 µg/ml streptomycin in the absence of insulin. The day after and prior to the test, cells were washed and incubated in the presence of the tested ingredients for 2 hours more in DMEM containing 5.5 mM D-glucose, 0.25 % BSA without insulin.

The glucose uptake was measured by incubation of the cells with radiolabelled 2-deoxy-D-[1,2 3H] glucose for 15 min. Glucose uptake was arrested by two washing steps in ice-cold PBS 1X. Then cells were solubilized in 0.1 N NaOH for 30 min. Cell associated radioactivity was then counted and protein quantification was determined using the coloric Lowry method.

Each ingredient was tested in triplicate.

Results are expressed in pmol glucose incorporated / min / mg protein and in % of control or basal condition (100 %).

A statistical analysis allowing multiple comparisons (one way ANOVA followed by a Dunnett's t test; *P < 0.05, **P < 0.01, ***P < 0.001 versus control condition) was carried out. All ingredients showing significant increase in glucose uptake compared to control or basal condition are considered active.

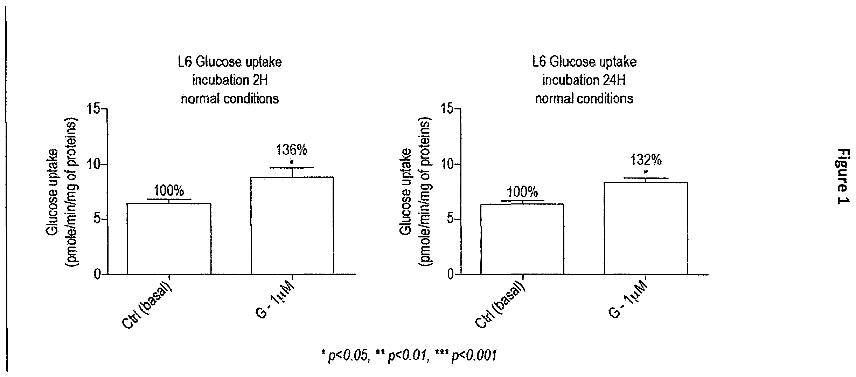

The results are shown in Figures 1 to 3.

As can be seen from figure 1, increases in glucose uptake were observed in L6 differentiated muscle cells with ingredient G after short term incubation (2 hrs) and long term incubation (24 hrs) in the absence of insulin.

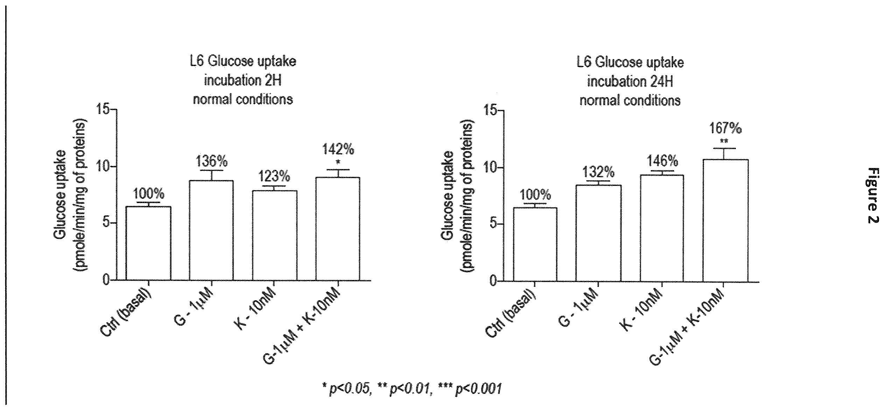

As can be seen from figure 2, increases in glucose uptake were observed in L6 differentiated muscle cells with the combination of ingredient G and ingredient K after short term incubation (2 hrs) and long term incubation (24 hrs) in the absence of insulin.

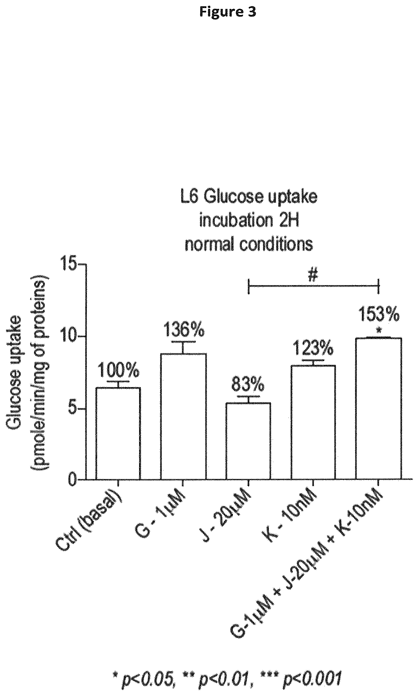

As can be seen from figure 3 increases in glucose uptake were observed in L6 differentiated muscle cell with the combination according to the present invention of ingredient G, K and ingredient J after short term incubation (2 hrs) in the absence of insulin.

## Claims

1. Composition comprising vitamin B2, vitamin D and zinc for use to treat or prevent Impaired Glucose Tolerance (IGT) wherein administration of said composition is not in-utero or through breastfeeding.

2. Composition for use according to claims 1 wherein said composition is administered enterally, more preferably orally.

3. Composition for use according to any one of claims 1 or 2 wherein said composition additionally contains at least one additional component selected from the group consisting of: myo-inositol, Vitamin B6, Vitamin B12, and/or a probiotic selected from Lactobacillus rhamnosus GG (CGMCC 1.3724) and/or Bifido Bacterium Lactis BB12 (CNCM I-3446).

4. Composition for use according to any one of claims 1 to 3 wherein the vitamin B2 is used in a concentration of 0.14 to 14 mg per daily dose, vitamin D is used in a concentration of 1.5 to 100 µg per daily dose and zinc is used in a concentration of 1.1 to 40 mg zinc per daily dose.

5. Composition for use according to any one of claims 1 to 4 wherein additionally myo-inositol if used is used in a concentration of 0.2 mg to 4 g per daily dose, vitamin B6 if used is used in a concentration of 0.14 to 14 mg per daily dose, vitamin B12 if used is used in a concentration of 0.26 to 26 µg per daily dose, and one or more probiotic if used is used in a concentration of 10e5 to 10e12 colony forming units (cfu) per daily dose.

6. Composition for use according to any one of claims 1 to 5 formulated to be administrated in the form of a nutritional product, a food product, a functional food product, a healthy ageing product, a dairy product, a nutritional supplement, a pharmaceutical formulation, a beverage product, a diet, or a pet food product.

7. A kit comprising:
(i) a composition of vitamin B2, vitamin D and zinc to be administered simultaneously or separately and (ii) instructions for dosing requirements for a subject for use to prevent or treat
Impaired Glucose Tolerance (IGT).

## Patentansprüche

1. Zusammensetzung, umfassend Vitamin B2, Vitamin D und Zink zur Verwendung, um eine Glucosetoleranzstörung (IGT) zu behandeln oder dieser vorzubeugen, wobei eine Verabreichung der Zusammensetzung nicht in-utero oder durch Stillen erfolgt.

2. Zusammensetzung zur Verwendung nach Anspruch 1, wobei die Zusammensetzung enteral, mehr bevorzugt oral, verabreicht wird.

3. Zusammensetzung zur Verwendung nach einem der Ansprüche 1 oder 2, wobei die Zusammensetzung zusätzlich mindestens eine zusätzliche Komponente enthält, die aus der Gruppe ausgewählt ist, bestehend aus: Myo-Inosit, Vitamin B6, Vitamin B12 und/oder einem Probiotikum, das aus Lactobacillus rhamnosus GG (CGMCC 1,3724) und/oder Bifido Bacterium Lactis 8812 (CNCM 1-3446) ausgewählt ist.

4. Zusammensetzung zur Verwendung nach einem der Ansprüche 1 bis 3, wobei das Vitamin B2 in einer Konzentration von 0,14 bis 14 mg pro Tagesdosis verwendet wird, Vitamin D in einer Konzentration von 1,5 bis 100 µg pro Tagesdosis verwendet wird und Zink in einer Konzentration von 1,1 bis 40 mg Zink pro Tagesdosis verwendet wird.

5. Zusammensetzung zur Verwendung nach einem der Ansprüche 1 bis 4, wobei zusätzlich Myo-Inosit, falls verwendet, in einer Konzentration von 0,2 mg bis 4 g pro Tagesdosis verwendet wird, Vitamin B6, falls verwendet, in einer Konzentration von 0,14 bis 14 mg pro Tagesdosis verwendet wird, Vitamin B12, falls verwendet, in einer Konzentration von 0,26 bis 26 µg pro Tagesdosis verwendet wird und ein oder mehrere Probiotika, falls verwendet, in einer Konzentration von 10e5 bis 10e12 koloniebildenden Einheiten (KBE) pro Tagesdosis verwendet wird.

6. Zusammensetzung zur Verwendung nach einem der Ansprüche 1 bis 5, die formuliert ist, um in Form eines Nährprodukts, eines Lebensmittelprodukts, eines funktionellen Lebensmittelprodukts, eines gesunden Alterungsprodukts, eines Milchprodukts, einer Nahrungsergänzung, einer pharmazeutischen Formulierung, eines Getränkeprodukts, einer Diät oder eines Haustiernahrungsprodukts verabreicht zu werden.

7. Kit, umfassend:
(i) eine Zusammensetzung von Vitamin B2, Vitamin D und Zink, die gleichzeitig oder getrennt verabreicht werden sollen, und (ii) Anweisungen für Dosierungsanforderungen für ein Subjekt zur Verwendung, um Glucosetoleranzstörung (IGT) zu verhindern oder diese zu behandeln.

## Revendications

1. Composition comprenant de la vitamine B2, de la vitamine D et du zinc pour une utilisation pour traiter ou prévenir une intolérance au glucose (IGT), dans laquelle l'administration de ladite composition n'est pas dans l'utérus ou par l'allaitement.

2. Composition pour utilisation selon la revendication 1, dans laquelle la composition est administrée par voie entérale, de préférence par voie orale.

3. Composition pour utilisation selon l'une quelconque des revendications 1 ou 2, dans laquelle ladite composition contient en outre au moins un composant supplémentaire choisi dans le groupe constitué : de myo-inositol, de vitamine B6, de vitamine B12, et/ou d'un probiotique choisi parmi Lactobacillus rhamnosus GG (CGMCC 1.3724) et/ou Bifide Bacterium Lactis BB12 (CNCM I-3446).

4. Composition pour utilisation selon l'une quelconque des revendications 1 à 3, dans laquelle la vitamine B2 est utilisée dans une concentration allant de 0,14 à 14 mg par dose journalière, de la vitamine D est utilisée dans une concentration allant de 1,5 à 100 µg par dose journalière et du zinc est utilisé dans une concentration allant de 1,1 à 40 mg de zinc par dose journalière.

5. Composition pour une utilisation selon l'une quelconque des revendications 1 à 4, dans laquelle, en outre, le myo-inositol, s'il est utilisé, est utilisé à une concentration allant de 0,2 mg à 4 g par dose journalière, la vitamine B6, si elle est utilisée, est utilisée à une concentration allant de 0,14 à 14 mg par dose journalière, la vitamine B12, si elle est utilisée, est utilisée à une concentration de 0,26 à 26 µg par dose journalière, et un ou plusieurs probiotiques, s'ils sont utilisés, sont utilisés à une concentration de 10e5 à 10e12 unités formatrices de colonies (UFC) par dose quotidienne.

6. Composition pour une utilisation selon l'une quelconque des revendications 1 à 5, formulée pour être administrée sous la forme d'un produit nutritionnel, d'un produit alimentaire, d'un produit alimentaire fonctionnel, d'un produit pour un vieillissement sain, d'un produit laitier, d'un complément nutritionnel, d'une formulation pharmaceutique, d'un produit de boisson, d'un régime alimentaire ou d'un produit alimentaire pour animal domestique.

7. Trousse comprenant :
(i) une composition de vitamine B2, de vitamine D et de zinc à administrer simultanément ou séparément et (ii) des instructions pour des exigences de dosage pour un sujet pour une utilisation pour empêcher ou traiter l'intolérance au glucose (IGT).
